# FASCICULE DE BREVET EUROPEEN

(11) **EP 2 723 383 B1**
(45) Date de publication et mention de la délivrance du brevet: **25.07.2018**
(21) Numéro de dépôt: 12728218.4
(22) Date de dépôt: 13.06.2012
(51) Int. Cl.: A61K 41/00, A61P 21/00

(54) **COMPOSITIONS HOMEOPATHIQUES POUR LE TRAITEMENT DES DOULEURS DU DOS, DES SCIATIQUES ET LOMBAIRES**
HOMÖPATHISCHE ZUSAMMENSETZUNGEN ZUR BEHANDLUNG VON RÜCKEN-, ISCHIAS-, ODER LUMBALSCHMERZ
HOMEOPATHIC COMPOSITIONS FOR TREATING BACK, SCIATIC, OR LUMBAR PAIN

(30) Priorité: 23.06.2011 BE 201100379
(43) Date de publication de la demande: 30.04.2014
(73) Titulaire: Mageren, Jean-Pierre, 7180 Seneffe (BE)
(72) Inventeur: Mageren, Jean-Pierre, 7180 Seneffe (BE)
(74) Mandataire: Office Kirkpatrick
(86) Numéro de dépôt international: PCT/EP2012/061160
(87) Numéro de publication internationale: WO 2012/175379

(56) Documents cités:
- US-A- 5 683 712
- Starbuck, J.: "Natural Cures for Back Pain" In: "More Ultimate Healing", 1 janvier 2007 (2007-01-01), Bottom Line Books, XP002672057, ISBN: 0-88723-457-7 Page 207, col. 2, paragraphe 3.
- Antony Cichoke: "Secrets of native american herbal medicine", 1 janvier 2001 (2001-01-01), Avery, Penguin Putnam, XP007920181, ISBN: 978-1-101-10025-7 Page 100, par. 1.
- Chernin Dennis: "The complete homeopathic resource for common illnesses,", 1 janvier 2006 (2006-01-01), NORTH ATLANTIC BOOKS, US, XP008147698, ISBN: 978-1-55643-608-6 page 109, Page 109, par. 3
- Anonymous: "Homeopathic and herbal products", Royal Pharmaceutical Society, 3 July 2014 (2014-07-03), XP055453919, Retrieved from the Internet: URL:http://www.rpharms.com/pharmacy-practi ce-resourcc/homeopathic-and-herbal-product ... [retrieved on 2018-02-23]

## Description

### Arrière-plan technique de l'invention

L'invention concerne une composition homéopathique destinée au traitement chronique des maux de dos et des membres inférieurs. L'invention traite en particulier les douleurs lombaires, telles que des lombalgies, lumbagos, hernies discales lombaires et des sciatiques. L'invention concerne plus particulièrement, le traitement des sciatiques.

Un rapport émis par l'ANAES (l'Agence nationale d'accréditation et d'évaluation en santé - FR) concernant les lombalgies chroniques (Diagnostic, prise en charge et suivi des malades atteints de lombalgie, décembre 2000) met en exergue combien les douleurs de dos peuvent être invalidantes et peuvent avoir des conséquences dans la vie quotidienne. Les recommandations concernant les méthodes de traitement utilisées peuvent également s'appliquer aux douleurs lombaires de manière générale.

Ce rapport détaille les différents traitements allopathiques utilisés et classés en fonction de l'intensité de la douleur du patient. Ce rapport concerne principalement l'utilisation d'antalgiques.

Les antalgiques de niveau I tel que le paracétamol peuvent être utilisés de façon ponctuelle pour soulager de la douleur, toutefois leur utilisation présente certains risques, en particulier en cas d'utilisation prolongée (risque d'ulcères notamment) et aucune étude n'aurait démontré l'efficacité du paracétamol dans cette utilisation.

Exemples d'antalgiques de niveau I (antalgiques non opioïdes) : Anti-inflammatoires non stéroïdiens, Acide acétylsalicylique, Paracétamol.

Les antalgiques de niveau II (opioïdes faibles) tel que les dérivés morphiniques (codéine, dextropropoxyphène...) et les antalgiques de niveau III (opioïdes forts ne pouvant être prescrits que de manière exceptionnelle) permettent de soulager la douleur toutefois leur utilisation comporte de nombreux effets indésirables. De plus, la durée du traitement doit être limitée dans le temps.

Exemples d'antalgiques de niveau II (antalgiques opioïdes faibles) : Codéine, Nalbuphine, Dextroproxyphène, Tramadol, Oxycodone, Buprénorphine.

Exemples d'antalgiques de niveau III (antalgiques opioïdes forts) : Morphine, Fentanyl, Hydromorphone.

Le rapport de l'ANAES détaille également l'utilisation à titre complémentaire de :
Myorelaxants (tétrazépam) : Ils sont utilisés en cas de contracture excessive et prolongée, Mais leurs effets sont incertains.

Antidépresseurs tricycliques : Leurs effets restent modestes.

L'utilisation de corticostéroïdes est quant à elle non recommandée selon ce rapport.

Il existe donc un besoin de traiter les douleurs de dos par une méthode alternative ne présentant pas les inconvénients ni les risques associés à l'utilisation ponctuelle, voir prolongée, des antalgiques, en particulier les risques de dépendance et autres effets secondaires non désirables.

L'homéopathie consiste en l'utilisation d'un ou de plusieurs agents actifs ou souches utilisés sous la forme d'une composition homéopathique. Les actifs se présentent généralement sous la forme d'une solution-mère ou teinture-mère laquelle est par la suite diluée dans l'eau ou l'alcool. Il existe plusieurs méthodes de dilution en homéopathie, les plus courantes étant les dilutions Hahnemanniennes décimales (DH ou X) ou centésimales (CH) ou les dilutions Korsakowiennes (K).

Par exemple, une solution de 6 DH correspond à 6 dilutions successives dans une proportion de 1 pour 9 d'une teinture-mère, généralement dans un mélange d'eau et d'alcool.

Après chaque étape de dilution, le mélange est agité d'une centaine de secousses ou succussions. L'ensemble de ces étapes est défini comme constituant la dynamisation de la solution. On trouve également des dilutions centésimales (proportion de 1 pour 99 à chaque dilution) et désignées par C, et des dilutions " milléniales " désignées par M (1 pour 999). Les techniques utilisées sont bien connues et sont, par exemple, décrites dans le Guide du préparateur en pharmacie (3eme édition, B. Charpentier et Al., éd. Masson).

De façon générale, les basses dilutions (4 ou 5 CH) sont recommandées dans le traitement des pathologies aiguës alors que les hautes dilutions (9 CH et au-delà) sont recommandées dans le traitement des pathologies chroniques.

La fréquence des prises dépend de la pathologie du patient. En cas de pathologie aiguë, les prises peuvent être rapprochées, tandis qu'elles seront plutôt espacées dans le cas de pathologies chroniques.

Par complexes homéopathiques, on entend des associations de traitements homéopathiques destinés à traiter un problème particulier.

Par souche homéopathique ou actif, on définit le produit, la substance ou la composition initiale permettant l'obtention d'un médicament homéopathique.

Par teinture-mère, on définit la solution initiale servant aux dilutions permettant l'obtention d'un médicament homéopathique. Le plus souvent, lorsque l'actif est d'origine végétale ou animale, la teinture-mère sert de souche.

Les médicaments homéopathiques se présentent, en général, sous la forme d'ampoules, de suppositoires, de formules en gouttes, de collyres, de granules, de triturations et de pommades. Selon l'invention, il est plus avantageux d'utiliser des granules car ces dernières permettent d'une part, un dosage aisé et, d'autre part, une grande praticité d'utilisation durant la journée.

Par traitement, il est entendu que l'invention permet d'obtenir une action curative et prophylactique des douleurs, en particulier des douleurs du dos et des membres inférieurs, telles que les sciatiques.

Par douleurs lombaires, on définit des douleurs ayant pour origine le bas du dos. Il peut s'agir de lombalgies, lumbagos, hernies discales lombaires et de sciatiques.

Une douleur est définie comme étant chronique lorsqu'elle persiste au-delà de ce qui est habituel pour la cause initiale présumée. Une douleur chronique est généralement définie comme étant une « expérience sensorielle et émotionnelle désagréable, liée à une lésion tissulaire existante ou potentielle, ou décrite en termes évoquant une telle lésion » (« Douleur chronique : reconnaître le syndrome douloureux chronique, l'évaluer et orienter le patient », Haute autorité de Santé, 2008, www.has-sante.fr).

La douleur est ce que la personne ressent, que la cause en soit identifiée ou non.

La douleur chronique prise en compte dans ces recommandations est un syndrome multidimensionnel exprimé par la personne qui en est atteinte. Il y a douleur chronique, quelles que soient sa topographie et son intensité, lorsque la douleur présente plusieurs des caractéristiques suivantes :
∘ persistance ou récurrence, durant au-delà de ce qui est habituel pour la cause initiale présumée, notamment si la douleur évolue depuis plus de 3 mois ;
∘ réponse insuffisante au traitement ;
∘ détérioration significative et progressive du fait de la douleur, des capacités fonctionnelles et relationnelles du patient dans ses activités de la vie journalière, au domicile comme à l'école ou au travail.

Ces douleurs du dos peuvent avoir différents facteurs déclenchant et leur origine peut être multiple : un problème osseux, articulaire, discal, ligamentaire et/ou musculaire.

Les sciatiques ont pour origine des lésions de la colonne vertébrale et des disques intervertébraux qui la composent. Les disques intervertébraux sont sujet au vieillissement, ce qui diminue leur élasticité et les fragilise d'autant. Ceci peut avoir pour conséquence une compression du nerf sciatique entraînant de vives douleurs au niveau des cuisses et va jusqu'à s'étendre le long de la jambe, au niveau du mollet, voire jusqu'au pied.

Les lombalgies ou mal de dos sont pour la plupart sans gravité et la douleur se résorbe spontanément après un certain temps. Dans certain cas, le mal de dos peut être chronique c'est-à-dire lorsqu'il perdure plus de 3 mois malgré les traitements.

La hernie discale apparaît lorsque le disque est altéré au point d'appuyer en permanence sur le nerf ce qui provoque une douleur intense et vive.

Il est connu de traiter par homéopathie les douleurs du dos et les sciatiques, en particulier l'ouvrage "AppliedHomeopathy" (R. Jacobs et M. E. Pinkerson. HomeopathyPress, Santa Monica, Calif., 1983) donne un exemple de composition homéopathique (Belladonna, Spigelia, Mag.phos. et de Cimifuga dont le facteur de dilution est de 6 X) pour le traitement des sciatiques.

L'ouvrage " Vademecum de la prescription en homéopathie " (A. Horvilleur, Ed. Masson, ISBN 2-294-08111-0) décrit également un traitement par homéopathie des lombalgies ; granules comprenant *Bryonia Alba* 5 CH, *Kalium Carbonicum* 5 CH, *NuxVomica* 5 CH, pris 3 fois/jour jusqu'à l'amélioration de l'état du patient.

Ce document décrit également différents composés homéopathiques couramment prescrits dans le traitement des douleurs lombaires, en particulier, AesculusHippocastanum, BerberisVulgaris, Bryonia Alba, Colocynthis, Dulcamara, Kalium Carbonicum, Natrum Muriaticum, NuxVomica, RhusToxicodendron, SepiaOfficinalis, ActaeaRacemosa, CalcareaFluorica, CalcareaPhosphorica, Causticum, HeloniasDioica, HypericumPerforatum, LycopodiumClavatum, Phosphorus, Pulsatilla, RutaGraveolens, Silicea, Sulfur.

Certaines souches (NuxVomica, RutaGraveolens...) sont également prescrites pour traiter des sciatiques.

Le " Traité d'homéopathie " (C. Gaucher, J-M Chabanne, Ed. Masson, ISBN 2-294-00703-4) décrit de nombreux composés homéopathiques pour le traitement des maux de dos, les lombalgies et les sciatiques. Le document mentionne, par exemple, l'utilisation de Medorrhinum et de Dioscorea pour le traitement des sciatiques. Le document mentionne également Radium Bromatum, CalcareaCarbonica, CalcareaPhosphorica, Natrum Muriaticum, Mandragora, Ferrum Metallicum, Senecio comme étant des composés efficaces pour le traitement de lombalgies et de douleurs lombaires.

Plusieurs ouvrages mentionnent de nombreuses souches sont le " Répertoire homéopathique de Kent " (MMI Ed.-Masson, 2001, ISBN : 2-84650-002-9, ISSN : 1292-4873) contient par exemple, une liste non exhaustive d'actifs agissant au niveau de la zone lombaire. Parmi ceux-ci figurent: Acon., Aesc., Aeth., Agar., All.-c., All.-s., Aloe, Alum., Ambr., Am.-c., Am.-m., Anag. , Ang., Ant-Ox, Apoc., Arg-m, Arg-n., Arn., Ars., Arund., Asaf., Aur., Aur.-m., Bar.-c., Bell., Berb., Bor., Brom., Bry., Bufo, Calad., Calc., Cale.-p., Camph., Canth., Caps., Carb.-ac., Carb-an., Carb.-s., Carb.-v., Caust., Cham., Chel., Chin., Chin.-s., Cic., Cimic., Cimx., Cina., Cinnb., Clem., Cob., Cocc., Coc.-c., Colch., Coloc., Con., Conv., Cop., Corn, Cor-r., Cupr.-ar., Cur., Cycl., Dig., Dios., Dulc., Elaps., Eup.-per., Fago., Ferr., Ferr.-ar., Ferr.-i, Ferr.-p., Fl.-ac., Gamb., Graph., Grat., Ham., Hell., Hep., Hura., Hydrc., Hyos., Ign., Indg., Iris, Jarr.,Nux-v, Kali.-br., Kali.-c., Kali.-i., Kali.-n., Kreos., Lac.-c., Lach., Lact., Led., Lept.,Lil-t, Lob., Lyc., lyss., Mag.-c., Mag.-m., Mag.-s., Med., Meli., Meny., Merc., Merc.-Oci., Naja, Nat.-a., Nat.-c., Nat-m., Nat.-s., nicc., Nux-m., Nux-v, Osm., Ox-Ac., Ph.-ac., Phel., Phos., Phys., Pic-ac., Plan., Plat., Plb., Prun., Ptel., Psor., Puls., Ran.-s., Rhod., Rhus-t., Ruta, Sabad, Sabin, Sang., Sep., Sil., Spong., Staph., Stront., Stry., Sul.-ac., Sul.-i., Sulph., Tab., Thuj., Vib., Zinc.

Le répertoire homéopathique de Kent répertorie aussi un certain nombre d'ingrédients permettant de traiter efficacement des sciatiques. Parmi les ingrédients figurent : Agar., Am.-m., Anan., arg.-n., Arn., Ars., Asar., Bar-c., Bapt., Bell., Berb., Bry., Calc., Caps., Carb.-s., Card.-m., Caust., Cham., Chel., Chin.-s., Cimic., Cocc., Coc.-c., Coff., Coloc., Dios., Dros., Elat., Eup.-pur., Ferr., Gels., Gnaph., Guai., Hyper., Ign., Indg., Iris, Kali.-bi., Kali.-br., Kali.-c., Kali.-i., Kali.-p., Lac.-c., Lach., Led., Lyc., Lyss., Mag.-p., Merc., Menv., Meny., Mez., Nat.-a., Nat.-m., Nat.-s., Nit.-ac., Nux.-m., Nux.-v., Ol.-j., Pall., Petr., Ph.-ac., Phos., Phyt., Plan., Plat., Plb., Podo., Psor., Puls., Ran.-b., Rhod., Rhus.-t., Ruta, Sal.-ac., Sep., Sil., Staph., Still., Stram., Sulph., Syph., Tell., Ter., thuj., Valer., Verat., Xan., Zinc.

Par ailleurs, de nombreuses souches sont utilisées en homéopathie pour leurs effets anti-inflammatoires. On peut citer, par exemple, Aconitum, Aconitum nap, Arnica, Bryonia, Eupatorium perf, HypericumRutaSymphytum (Homéopathie pratique, Dr. C. Binet, Ed. Dangles, p. 30, 93, 159, 176) Apis, Harpagophytum, Salix, Harpagophytum, Hamamelis, Fraxinus, Tilia, Callunna. Ces ingrédients permettent de soulager certains types de douleurs inflammatoires. Toutefois, leur efficacité ne permet pas d'obtenir une action spécifique pour le traitement et/ou la prévention des douleurs lombaires.

La demande US 2005/0100513 concerne une composition homéopathique comprenant Staphysagria et/ou Spigelia. Cette composition est destinée à traiter l'addiction à la nicotine et n'a pas été décrit par ses inventeurs comme présentant un effet pour le traitement des maux de dos. Cette demande concerne de ce fait un domaine éloigné de celui de l'invention.

La demande WO 2010/57295 décrit une composition homéopathique comprenant Cuprum et Spigelia pour le traitement des douleurs névropathiques et concerne là aussi un domaine éloigné de l'invention.

Les brevets EP 1 236 466 et US 5,683,712 mentionnent une composition homéopathique comprenant Spigelia décrit dans " AppliedHomeopathy " R ; Jacobs, M. E. Pinkerson, HomeopathyPress, Santa Monica, Calif., 1983). Cette composition permet de traiter les douleurs sciatiques. Toutefois la composition décrite consiste en une combinaison spécifique de 4 ingrédients actifs (Belladonna 6X, Spigelia 6X, Mag. Phos. 6X et Cimifuga 6X) qui ne permettrait pas d'avoir un effet préventif, cette composition ne comportant pas d'agent homéopathique anti-inflammatoire susceptible d'induire une stimulation corticosurrénale.

« More Ultimate Healing », (2007-01-01), Bottom Line Books décrit l'utilisation en homéopathie de Cuprum Metallicum pour le traitement du mal de dos.« The completehomeopaticresource for commonillnesses », (2006-01-01), Denis Chermin, Northatlantic books, Berkley CA. celle de Staphysagria pour le traitement des lombalgies. Toutefois ces compositions sont inefficaces dans le traitement de douleurs lombaires chroniques et ne permet pas de prévenir l'apparition de douleur de dos.

« Secrets of native americanherbalmedicine », (2001-01-01), Avery, Penguin Putnam, décrit l'utilisation de Ribes Nigrumpour soulager le mal de dos, toutefois, il n'est pas décrit de traitement homéopathique.

L'un des objets de l'invention concerne une composition homéopathique permettant d'obtenir une action prophylactique et préventive pour le traitement des douleurs lombaires telles que les lombalgies, lumbagos, hernies discales et les sciatiques, en particulier de douleurs chroniques.

Un autre objet de l'invention concerne une composition homéopathique particulièrement efficace pour soulager les douleurs lombaires, notamment des douleurs lombaires chroniques.

Un autre objet de l'invention concerne une composition homéopathique particulièrement efficace pour soulager les sciatiques.

Un autre objet de l'invention concerne une composition homéopathique utilisée de manière préventive pour éviter les douleurs lombaires.

Un autre objet de l'invention concerne une composition homéopathique utilisée de manière préventive pour éviter les sciatiques.

Ainsi, l'invention de la présente demande concerne la composition homéopathique de la revendication 1 et les médicaments homéopathiques de la revendication 6.

### Description des figures

La Figure 1 est un graphique illustrant l'évolution de la douleur lombaire chronique sur une période de 60 jours sur 7 sujets traités par homéopathie.
La Figure 2 illustre l'évolution de la douleur sur une période de 24 heures sur 9 sujets traités par homéopathie.

### Description détaillée de l'invention

A la connaissance de l'inventeur, il n'existe pas de compositions homéopathiques comprenant Cuprum, Staphysagria et Ribes Nigrum permettant d'obtenir une action préventive et curative des douleurs de dos et/ou des membres inférieurs tels que les lombalgies et les hernies discales, notamment des douleurs chroniques.

Les caractéristiques des souches utilisées sont référencées de façon exhaustive par exemple par " The HomoeopathicPharmacopoeia of the United States " ou HPUS et par d'autres banques de données. Dans l'ouvrage " Homéopathie pratique, Dr. C. Binet, Ed. Dangles " sont décrits :

### CUPRUM (" Homéopathie pratique " p. 70)

Il s'agit de cuivre sous sa forme métallique. Ce produit est utilisé en homéopathie pour traiter différentes pathologies dont les crampes et les contractions musculaires telles que les spasmes.

### RIBES NIGRUM (" Homéopathie pratique " p. 156)

Cassis, grossularinacées, lequel est connu pour ses propriétés diurétiques et dépuratives. Ribes Nigrum est surtout utilisé lors d'allergies, son utilisation permettant notamment une stimulation corticosurrénale induisant la sécrétion d'hormones anti-inflammatoires.

### SPIGELIA (" Homéopathie pratique " p. 170)

Spigélieanthelminique ou Brinvillière d'Amérique est une plante connue utilisée comme traitement des palpitations cardiaques, de l'angine de poitrine, de l'endocardite et des affections valvulaires.

Spigelia est également connue comme particulièrement efficace dans le traitement de la sclérite rhumatismale.

Une autre utilisation de Spigelia concerne le traitement de tout trouble lié à la présence de parasites intestinaux.

### STAPHYSAGRIA (" Homéopathie pratique " p. 172)

Aussi connue comme herbe aux poux, Staphysagria est une renonculacée utilisée en homéopathie pour traiter du prurit urinaire et des organes génito-urinaire avec alternance de troubles cutanés et des rhumatismes.

Les compositions homéopathiques utilisées jusqu'à présent pour traiter des douleurs de dos se sont avérées peu efficace. Or, l'inventeur à constater que la combinaison de Cuprum, Ribes Nigrum, et Staphysagria dans une composition homéopathique permet d'améliorer de façon significative le traitement de ces douleurs.

L'inventeur a formulé et développé une composition homéopathique particulièrement avantageuse car elle permet d'obtenir un effet préventif en plus d'un effet curatif et ne présente pas les effets secondaires attendus par l'utilisation des actifs pris séparément. Par ailleurs, ladite composition présente un effet remarquable pour soulager des douleurs de dos chroniques.

L'utilisation de Staphysagria est connue en homéopathie comme étant un décontractant musculaire et peut également être employée pour soulager des douleurs musculaires. Staphysagria est également utilisée dans de nombreuses compositions homéopathiques destinées à soulager les maux de dos. Staphysagria permet de soulager et d'apaiser les douleurs, et permettrait d'obtenir, lors de maux de dos, une action localisée aux membres inférieurs et aux dos dans la zone lombaire.

Ribes Nigrum est généralement utilisé dans les compositions homéopathiques afin de stimuler la libération de cortisone produite par le corps. Obtenant ainsi un effet anti-inflammatoire, contrairement à ce que l'on obtient par l'utilisation de Staphysagria. Une description plus complète des propriétés de Ribes Nigrum peut être obtenue dans l'ouvrage " Homéopathie pratique " (Dr. C. Binet, Ed. Dangles, p. 156). A la suite de l'utilisation de Ribes Nigrum, il est également fréquent de constater l'apparition de crampes musculaires.

L'inventeur a constaté que l'addition de Cuprum Metallicum n'affecte en rien l'efficacité de Ribes Nigrum et de Staphysagria pour le traitement des maux de dos et permettrait, en plus, d'éliminer ou de réduire sensiblement l'apparition des crampes musculaires ainsi que toute autre forme de contractures.

Par ailleurs, l'utilisation de cette composition homéopathique permet d'obtenir un effet préventif et prophylactique particulièrement efficace sur l'apparition des douleurs lombaires. Sans être lié à une quelconque hypothèse scientifique, il semblerait que l'effet prophylactique soit lié à l'utilisation de Ribes Nigrum qui permet la libération de cortisone dans le corps et qui préviendrait celui-ci contre d'éventuelles douleurs.

D'autre part, l'ajout de Spigelia dans la composition apporte un avantage supplémentaire. Spigelia est un actif utilisé en homéopathie. Lorsqu'une personne arrête soudainement un traitement par allopathie, en particulier la prise d'un antidouleur ou d'un anti-inflammatoire, il est fréquent qu'un état de manque survienne, il peut par exemple exister une dépendance à des produits tel que la codéine. L'utilisation de Spigelia tend à pallier et calmer cet état de manque et à permettre un sevrage. Il est donc avantageux d'utiliser Spigelia en début de traitement notamment pour des patients ayant eu recours à un traitement allopathique.

Une composition homéopathique telle que développée comprenant Cuprum Metallicum, Staphysagria et Ribes Nigrum est donc particulièrement efficace dans la prévention, le soulagement et le traitement de maux de dos.

De préférence, la composition homéopathique est utilisée en dilution comprise entre 5 et 7 CH (centésimale Hahnemannienne). La composition homéopathique comprend avantageusement, Cuprum Metallicum, et Ribes Nigrum en dilution à 5 CH et Staphysagria en dilution à 7 CH. De façon plus avantageuse, la composition homéopathique contient également Spigelia en dilution comprise entre 5 et 7 CH et de préférence de 5 CH.

L'aspect préventif est obtenu par la prise de 2 à 3 granulés au moins une heure, de préférence entre 1 et 8 heures, avant d'effectuer un effort physique susceptible d'engendrer un mal de dos, ce qui permet d'éviter l'apparition de douleurs lombaires et/ou sciatiques.

Le traitement des douleurs lombaires est de préférence obtenu par la prise de 2 granulés 3 fois par jour. Ce traitement peut être occasionnel, c'est-à-dire après l'apparition de douleurs lombaires ou suite à un effort. Le traitement peut également être prolongé, par exemple dans le cas de douleurs chroniques. Dans ce cas, le traitement préférentiel consiste en la prise de 2 granulés 3 fois par jour pendant une période de 3 à 6 mois. Suite à ce traitement, une amélioration de l'état des malades est observée. Un rétablissement complet du malade pouvant également être observé si ce traitement est maintenu sur une durée supplémentaire de 3 à 6 mois.

### Exemples :

### Mesure de la douleur

La douleur ressentie est évaluée par le patient lui-même sur une échelle d'évaluation sous forme d'échelle verbale simple (EVS) comprenant 10 graduations, chacune correspondant à un adjectif qui qualifie la douleur selon son intensité: aucune (0), légère (2), gênante (4), forte (6), atroce (8), maximale imaginable (10).

Outre le témoignage oral du patient, l'observation faite par le médecin est prise en considération.

Les exemples 1-6 correspondent à des patients ayant utilisés une composition homéopathique comprenant : Cuprum Metallicum (Cupr. Metall.)5 CH, Staphysagria (Staph.) 7 CH, Ribes Nigrum 5 CH, Spigelia 5 CH.

Les exemples 7 et 8 correspondent à des patients ayant utilisés une composition homéopathique comprenant : Cuprum Metallicum 5 CH, Staphysagria 7 CH et Ribes Nigrum 5 CH.

La composition homéopathique peut être obtenue par dilution d'une teinture-mère, dans un solvant approprié, de préférence de l'eau et/ou de l'alcool. Chacune de ces étapes de dilution étant suivie d'une étape de succussion qui consiste en une agitation vigoureuse de la solution par au moins 100 secousses. Le mélange est ensuite diffusé, sur des granules inertes.

Les souches utilisées par la présente invention sont commercialisées à l'état dilué par la Société BOIRON sous les références SPIGELIA(HAB), STAPHYSAGRIA (PHFR), RIBES NIGRUM GEMMAE MG (HAB), CUPRUM METALLICUM 5 CH.

Les granulés inertes ont été fournis par la société Debofar sous la référence PF125 (85/15) 4 mm.

Selon le mode de réalisation préféré, le mélange comprend 1 ml de Ribes Nigrum 5 CH à 70 deg, 1 ml de Staphysagria 7 CH à 70 deg, 1 ml de Spigelia 5 CH à 70 deg et 1 ml de Cuprum Metallicum 5 CH à 70 deg. Le mélange est ensuite diffusé, sur 60 g de granules neutres de saccharose ou de glucose préalablement calibrés.

Résultats obtenus sur plusieurs patients :

**Tableau 1**

| Cas | Traitement | Durée du traitement | Effets / observations |
|---|---|---|---|
| 1. Sciatique | 2 granulés 3 fois par jours | 3 mois | Disparition complète des douleurs |
| 2. Courbatures et posture courbée | 2 granulés 3 fois par jours En complément de la prise d'antidouleur et d'anti-inflammatoire | 1 mois | A permis de diminuer la prise des antidouleurs et des anti-inflammatoires Redressement de la posture |
| Suite du cas 2. | | 3 mois | Rétablissement total |
| 3. Sciatique | 2 granulés 3 fois par jours En complément de la prise d'antidouleur et d'anti-inflammatoire | 1 mois | Rétablissement total |
| 4. Douleur de dos chronique | 3 granulés 3 fois par jours | 1 à 3 mois | A permis de diminuer la prise des antidouleurs et des anti-inflammatoires |
| 5. Sciatique | 3 granulés 3 fois par jour | 1 mois | A permis de réduire la prise d'antidouleur et anti-inflammatoire |
| Suite du cas 5. | 3 granulés 3 fois par jour | 6 mois | Suppression de la prise d'antidouleur et anti-inflammatoire. |
| 6. Sciatique | 2 granulés 3 fois par jours | 15 jours | Rétablissement total |
| 7. Douleurs suite à un effort physique | 3 granulés immédiatement puis toutes les 2 heures jusqu'à la disparition de la douleur | Quelques heures | Disparition effective de la douleur après quelques heures |
| 8. Effet préventif | 2 à 3 granulés pris 1 à 8 heures avant d'effectuer un effort | | Pas de douleurs durant les 8 heures qui suivent la prise des granulés |

Cas 1 : Concerne un homme de 50 ans accidenté et alité à la suite d'une collision en voiture et souffrant de sciatique.

Une amélioration de son état a été constatée après 10 jours de traitement, une disparition complète des douleurs après 3 mois.

Cas 2 : Concerne un fermier de 50 ans souffrant de lombalgie chronique ayant une posture courbée et prenant 1 à 3 comprimés d'anti-inflammatoire (Arcoxia) par semaine. Le traitement a consisté en la prise de 2 granulés 3 fois par jours pendant un mois, en complément de la prise d'anti-inflammatoire. Par la suite, la prise d'antidouleur et d'anti-inflammatoire a été progressivement réduite pour être complètement supprimée. Après 1 mois, on a constaté un redressement de la posture. Après 3 mois, il a été constaté un redressement complet.

Cas 3 : Concerne un homme de 53 ans souffrant d'une sciatique due à son activité professionnelle (gardien de salle de sport) qui le contraint à rester debout. Le traitement a consisté en la prise de 2 granulés 3 fois par jours, en complément de la prise d'antidouleur et d'anti-inflammatoire, après un mois de traitement, la prise d'antidouleur et d'anti-inflammatoire a pu être complètement supprimée et on a constaté une disparition complète des douleurs.

Cas 4 : Concerne une jeune fille de 25 ans qui souffre de douleur chronique au dos. Elle remplace la prise d'antidouleur et anti-inflammatoire (Dafalgan) par 3 granulés de la composition homéopathique à la demande.

Cas 5 : Concerne un homme de 55 ans souffrant de sciatique. La prise de 3 granulés 3 fois par jour pendant 1 mois a permis de réduire la prise d'antidouleur et anti-inflammatoire (Dafalgan codéine et Feldene) et en prolongeant la prise 3 granulés 3 fois par jour pendant 6 mois, a permis d'arrêter complètement la prise d'antidouleur et anti-inflammatoire.

Cas 6 : Concerne une femme d'environ cinquante ans, ayant une sciatique suite à une agression physique. La prise de 2 granulés 3 fois par jours pendant 15 jours a permis de faire disparaître la douleur.

Cas 7 : Concerne de jeunes travailleurs de 20-25ans travaillant comme manoeuvre dans le bâtiment et souffrant de maux de dos suite à des efforts physiques. La prise de 3 granulés selon l'invention a permis de soulager puis d'éliminer la douleur.

Cas 8 : Concerne un traitement préventif à l'apparition des maux de dos sur deux sujets âgés de 61 et de 23 ans travaillant sur un chantier. La prise de 3 granulés selon l'invention chaque matin avant les travaux a permis de prévenir l'apparition de maux de dos sur la période de 3 ans correspondant à la durée du chantier.

### Douleurs lombaires chroniques

L'étude a été réalisée sur une période de deux ans. Un groupe de 7 personnes, hommes et femmes, âgés de 40 à 65 ans et souffrant de douleurs lombaires chroniques ont été choisis après vérification des conditions suivantes:
- vérification par des examens médicaux traditionnels notamment par prise de sang et par radios qu'il s'agissait bien de douleurs lombaires et non pas de cas de rhumatisme, arthrite ou arthrose.
- Vérification que les douleurs lombaires datent de plus de 3 mois et que la prise de médicaments anti-inflammatoires est restée sans effet.
- Vérification des traitements médicaux pour écarter tout patient ne souffrant pas seulement de douleurs lombaires chroniques mais souffrant également de rhumatisme, arthrite ou arthrose en association à des douleurs lombaires chroniques.

L'état initial des patients a été évalué selon la méthode de l'échelle EVS, qui consiste en ce que le patient se situe lui-même sur une échelle allant de 0 à 10, 0 signifiant l'absence de douleur et 10 signifiant une douleur insupportable. L'évaluation comprenait les questions suivantes:
- Quand vous êtes dans un moment de grande douleur, êtes-vous capable de faire toute activité physique ?
- Quand vous êtes dans un moment de grande douleur, êtes-vous capable de faire toute activité mentale ?
- Vous sentez-vous capable, chaque jour au lever, de travailler ?

Afin d'évaluer l'efficacité de chaque actif, un groupe témoin constitué de 3 individus a reçu 2 granulés homéopathiques trois fois par jour un quart d'heure avant les repas pendant 1 mois. Chacune de ces personnes étant uniquement traitée par des granulés comprenant un complexe composé soit de Cuprum Metallicum 5CH, soit de Staphysagria 7CH ou soit de Ribes Nigrum 5CH.

Les 4 autres personnes participant à l'évaluation du complexe selon l'invention ont reçu un médicament homéopathique comprenant Cuprum Metallicum 5 CH, Staphysagria 7 CH et Ribes Nigrum 5CH. La posologie étant identique à celle du groupe témoin à savoir la prise trois fois par jour et pendant 1 mois de 2 granulés homéopathique.

Une évaluation de la douleur ressentie par chaque patient a été mesurée à 10, 20 et 30 jours de traitement, au lever. Les résultats de ces tests sont résumés dans le

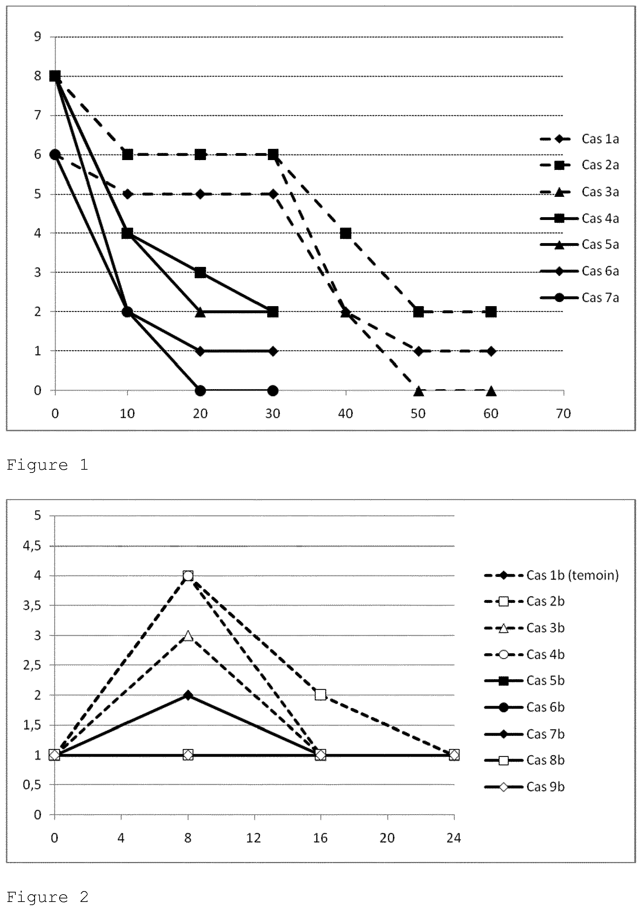

Tableau **2** ci-après et illustré par le graphique de la Figure 1.

**Tableau 2 : Mesure de la douleur sur une échelle EVS (0 - 10).**

| Cas | la | 2a | 3a | 4a | 5a | 6a | 7a |
|---|---|---|---|---|---|---|---|
| Actifs | Cupr. Metall. 5 CH | Staph. 7 CH | Ribes Nigrum 5 CH | Cupr.Metall. 5 CH, Staph. 7 CH et Ribes Nigrum 5 CH | Cupr. Metall. 5 CH, Staph 7 CH et Ribes Nigrum 5 CH | Cupr.Metall . 5 CH, Staph. 7 CH et Ribes Nigrum 5 CH | Cupr. Metall. 5 CH, Staph. 7 CH et Ribes Nigrum 5 CH |
| Avant la prise de médicament | 6 | 8 | 8 | 8 | 8 | 8 | 6 |
| Après 10 jours | 5 | 6 | 6 | 4 | 4 | 2 | 2 |
| Après 20 jours | 5 | 6 | 6 | 3 | 2 | 1 | 0 |
| Après 30 jours | 5 | 6 | 6 | 2 | 2 | 1 | 0 |
| Actifs | Cupr. Metall. 5 CH, Staph. 7 CH et Ribes. Nigrum 5 CH | Cupr. Metall. 5 CH, Staph. 7 CH et Ribes Nigrum 5 CH | Cupr. Metall. 5 CH, Staph. 7 CH et Ribes Nigrum 5 CH | | | | |
| Après 40 jours | 2 | 4 | 2 | | | | |
| Après 50 jours | 1 | 2 | 0 | | | | |
| Après 60 jours | 1 | 2 | 0 | | | | |

Sur une période de 1 mois on constate que pour les personnes du groupe témoin (cas 1a, 2a et 3a) ayant suivi un traitement par l'un des actifs présentent peu voir pas d'amélioration au niveau de l'intensité perçue de leur douleur (Figure 1).

Les personnes (cas 4a, 5a, 6a, 7a) ayant reçu un traitement selon l'invention ont ressenti une diminution significative de l'intensité de la douleur perçue (Figure 1) .

Il a donc été confirmé que la prise de médicament composé d'un complexe selon l'invention permet de soulager rapidement les patients de leurs douleurs chroniques avec des effets observés dès le début du traitement.

Dans une étape ultérieure, le groupe témoin ayant reçu les granulés comprenant l'un des actifs ont été traités par une composition homéopathique selon l'invention (cas 1a - 3a). Le traitement a consisté en la prise de 2 granulés trois fois par jour pendant 1 mois.

Il a été confirmé que la prise de médicament composé d'un complexe selon l'invention permet de soulager les patients de leurs douleurs chroniques avec des effets observés dès le début de la prise du médicament comprenant l'actif.

Le traitement de ces patients par un médicament homéopathique comprenant pour complexe Cuprum Metallicum 5 CH, Staphysagria 7 CH et Ribes Nigrum 5 CH, a permis de diminuer de façon significative la douleur chronique.

### Evaluation du médicament dans la prévention des douleurs lombaires.

Une étude a été réalisée afin de mesurer les effets préventifs du médicament homéopathique sur l'apparition de maux de dos.

Cette étude a été réalisée sur une période de deux ans et a été effectuée à partir d'un groupe composé de 9 personnes âgés de 18 à 65 ans travaillant en tant que ouvrier ou manoeuvre dans le bâtiment, magasinier, chauffeur livreur, mécanicien automobile, chacune de ces personnes souffrant de douleurs lombaires.

Les patients souffrant de douleurs lombaires dues à un effort ont été sélectionnées après vérification des conditions suivantes:
- Vérification des traitements médicaux afin d'écarter tout patient ne souffrant pas uniquement de douleurs lombaires mais souffrant également de rhumatisme, arthrite ou arthrose.
- Vérification que les douleurs lombaires soient associées à un effort de travail répondant à un cycle de 24 heures comprenant 3 périodes de 8 heures.
- Elimination des cas de rhumatisme, arthrite ou arthrose grâce à des examens médicaux traditionnels notamment par une prise de sang ou par radios.

L'état initial des patients a été évalué selon la méthode EVS, sur une échelle des douleurs graduée de 0 à 10 ; 0 signifiant l'absence de douleur et 10 indiquant la douleur maximale imaginable. L'évaluation comprenant les questions suivantes:
- « Au lever, quelle est l'intensité de la douleur sur une échelle EVS graduée de 0 à 10? »
- « Après une journée d'activité, quelle est l'intensité de la douleur sur une échelle EVS graduée de 0 à 10?»
- « Au coucher, quelles est l'intensité de la douleur sur une échelle EVS graduée de 0 à 10?»

Chaque jour, le patient reporte la mesure des douleurs lombaires sur l'échelle EVS après le lever; après sa journée de travail et avant son coucher. Les valeurs moyennes de ces mesures sont reportées à la Figure 2.

Les cas 1b- 9b ont été traités de façon préventive par la prise de 2 granulés homéopathiques une fois par jour, pendant une semaine, 1 heure avant le début de leur activité professionnelle.

Les cas 1b à 4b constituent un groupe d'individu témoin utilisant respectivement des granulés ne comprenant pas d'actif, des granulés composés de Cuprum Metallicum 5 CH, des granulés composés de Staphysagria 7 CH, des granulés composés de Ribes Nigrum 5 CH.

Les cas 5b à 9b ont reçu des granulés de médicament homéopathique selon l'invention et composé de Cuprum Metallicum 5 CH, Staphysagria 7 CH et Ribes Nigrum 5 CH.

L'intensité de la douleur a ensuite été relevée à différente période de la journée. Les moyennes des résultats obtenus sur la semaine sont reportées au Tableau 3.

**Tableau 3**

| cas | 1b (témoin) | 2b | 3b | 4b | 5b | 6b | 7b | 8b | 9b |
|---|---|---|---|---|---|---|---|---|---|
| Actifs | Aucun | Cupr.Metall . 5 CH | Staph. 7 CH | Ribes Nigrum 5 CH | Cupr.Meta 115 CH, Staph. 7 CH et Ribes Nigrum 5 CH | Cupr. Metall . 5 CH, Staph. 7 CH et Ribes Nigrum 5 CH | Cupr. Metall . 5 CH, Staph. 7 CH et Ribes Nigrum 5 CH | Cupr. Metall . 5 CH, Staph. 7 CH et Ribes Nigrum 5 CH | Cupr. Metall. 5 CH, Staph. 7 CH et Ribes Nigrum 5 CH |
| Intensité de la douleur au moment du levé (valeur moyenne sur une semaine) | 1 | 1 | 1 | 1 | 1 | 1 | 1 | 1 | 1 |
| Intensité de la douleur 8 heures après le levé (valeur moyenne sur une semaine) | 4 | 4 | 3 | 4 | 1 | 1 | 2 | 1 | 1 |
| Intensité de la douleur 16 heures après le levé (valeur moyenne sur une semaine) | 2 | 2 | 1 | 1 | 1 | 1 | 1 | 1 | 1 |
| Intensité de la douleur 24 heures après le levé (valeur moyenne sur une semaine) | 1 | 1 | 1 | 1 | 1 | 1 | 1 | 1 | 1 |

### Évolution de la douleur

La Figure 2 indique l'évolution de la douleur sur une période de 24 heures à compter du lever de la personne.

La journée peut être divisée en trois périodes d'environ 8 heures à partir du lever, correspondant à 8 heures d'activité professionnelle, 8 heures de vie sociale et 8 heures de repos et de sommeil.

La personne commence généralement sa journée de travail sans douleurs lombaires. En effet, au lever, les douleurs lombaires suites à des efforts physiques sont nulles ou pratiquement nulles. Au cours de la journée les douleurs lombaires augmentent progressivement jusqu'à atteindre un niveau décrit comme étant gênant (cas 1b à 4b).

Pendant de la période de 8 heures suivant la période d'activité professionnelle, on observe une diminution progressive de l'intensité des douleurs qui tend jusqu'à la disparition complète de celle-ci dans la 3^{eme} période de 8 heures correspondant à la période de repos et de sommeil des patients.

Lors de ces tests, l'inventeur a constaté que l'utilisation de Cuprum Metallicum, Staphysagria ou Ribes Nigrum pris séparément ne permet pas d'obtenir un effet préventif sur l'apparition de maux de dos, la douleur perçue par les cas 2b -4b lors de leur période d'activité étant proche de celle perçu par le patient témoin (cas 1b).

Le complexe selon l'invention composé de Cuprum Metallicum, Staphysagria et de Ribes Nigrum permet donc bien d'obtenir un effet préventif pour les cas 5b à 9b qui s'observe par une absence d'augmentation de la douleur au cours de la journée.

## Revendications

1. Composition homéopathique comprenant : Cuprum Metallicum, Staphysagria et Ribes Nigrum **caractérisée par le fait que** la dilution des souches homéopathiques est de 5 CH pour Cuprum Metallicum, et Ribes Nigrum et 7 CH pour Staphysagria.

2. Composition homéopathique selon la revendication 1 comprenant en outre Spigelia à une dilution de 5 CH.

3. Composition homéopathique selon l'une des revendications 1 à 2, pour son application comme médicament à effet préventif des douleurs lombaires telles que lombalgies, lumbagos, hernies discales lombaires et sciatiques, **caractérisée en ce que** le médicament est utilisé au moins une heure, de préférence entre 1 et 8 heures, avant d'effectuer un effort susceptible d'entraîner une douleur des lombaires et/ou sciatique.

4. Composition homéopathique selon l'une des revendications 1 à 2, pour son application comme médicament permettant de soulager les douleurs telles que lombalgies, lumbagos, hernies discales lombaires et sciatiques, **caractérisée en ce que** le médicament soit pris 2 à 3 fois par jour lorsque lesdites douleurs apparaissent.

5. Composition homéopathique selon l'une des revendications 1 à 2 pour son application comme médicament permettant de traiter les douleurs lombaires chroniques telles que lombalgies, lumbagos, hernies discales lombaires et sciatiques, **caractérisée en ce que** le médicament soit pris 2 à 3 fois par jour pour une période prolongée de 3 à 6 mois.

6. Médicaments homéopathiques comprenant la composition homéopathique selon l'une des revendications 1 à 2 **caractérisé en ce que** le médicament est conditionné sous une des formes suivantes: ampoules, suppositoires, formules en gouttes, collyres, granules, triturations, pommades.

## Patentansprüche

1. Homöopathische Zusammensetzung, die *Cuprum Metallicum, Staphysagria* und *Ribes Nigrum* enthält, **dadurch gekennzeichnet, dass** die Potenzierung der homöopathischen Stämme für *Cuprum Metallicum* und *Ribes Nigrum* 5 CH und für *Staphysagria* 7 CH beträgt.

2. Homöopathische Zusammensetzung nach Anspruch 1, die außerdem *Spigelia* in einer Potenz von 5 CH enthält.

3. Homöopathische Zusammensetzung nach einem der Ansprüche 1 bis 2 zur Anwendung als Arzneimittel mit vorbeugender Wirkung gegen Schmerzen im Bereich der Lenden, wie bei Lumbalgien, Lumbago, lumbalen Bandscheibenvorfällen und Lumboischialgien, **dadurch gekennzeichnet, dass** das Arzneimittel mindestens eine Stunde, vorzugsweise zwischen 1 und 8 Stunden, bevor es zu einer Beanspruchung kommt, die möglicherweise Schmerzen im Bereich der Lenden und/oder Ischiasschmerzen verursacht, angewendet wird.

4. Homöopathische Zusammensetzung nach einem der Ansprüche 1 bis 2 zur Anwendung als Arzneimittel zur Linderung von Schmerzen, wie etwa bei Lumbalgien, Lumbago, lumbalen Bandscheibenvorfällen und Lumboischalgien, **dadurch gekennzeichnet, dass** das Arzneimittel 2- bis 3-mal täglich eingenommen wird, wenn die Schmerzen auftreten.

5. Homöopathische Zusammensetzung nach einem der Ansprüche 1 bis 2 zur Anwendung als Arzneimittel zur Behandlung von chronischen Schmerzen im Bereich der Lenden, wie etwa bei Lumbalgien, Lumbago, lumbalen Bandscheibenvorfällen und Lumboischalgien, **dadurch gekennzeichnet, dass** das Arzneimittel über einen längeren Zeitraum von 3 bis 6 Monaten 2- bis 3-mal täglich eingenommen wird.

6. Homöopathische Arzneimittel, die die homöopathische Zusammensetzung nach einem der Ansprüche 1 bis 2 umfassen, **dadurch gekennzeichnet, dass** das Arzneimittel in einer der folgenden Formen abgepackt ist: Ampullen, Suppositorien, Tropfenformulierungen, Augentropfen, Zuckerkügelchen, Triturationen, Salben.

## Claims

1. A homeopathic composition comprising Cuprum metallicum, Staphysagria and Ribes nigrum, **characterized in that** the dilution of homeopathic stocks is 5 CH for Cuprum metallicum and Ribes nigrum and 7 CH for Staphysagria.

2. The homeopathic composition as set forth in claim 1, further comprising Spigelia at a dilution of 5 CH.

3. The homeopathic composition as set forth in any one of claims 1 to 2 for is application as a medicament for preventing lumbar pain such as lumbago, lumbago, herniated lumbar disc, and sciatica, **characterized in that** the medicament is used at least one hour, preferably between 1 and 8 hours, before performing an activity that is likely to cause pain of the lumbar pain and/or sciatica.

4. The homeopathic composition as set forth in any one of claims 1 to 2 for application as a medicament for relieving pain such as lumbago, lumbago, herniated lumbar disc, and sciatica, **characterized in that** the medicament is taken 2 to 3 times a day when the appears.

5. The homeopathic composition as set forth in any one of claims 1 to 2 for application as a medicament for treating chronic lumbar pain such as lumbago, lumbago, herniated lumbar disc, and sciatica, **characterized in that** the medicament is taken 2 to 3 times per day for an extended period of 3 to 6 months.

6. Homeopathic medicaments comprising the homeopathic composition as set forth in any one of claims 1 to 2, **characterized in that** the medicament is packaged in one of the following forms: ampoules, suppositories, formulations for drops, eye drops, pellets, triturations, ointments.
